# EUROPÄISCHE PATENTANMELDUNG

(11) **EP 3 213 716 A1**
(43) Veröffentlichungstag der Anmeldung: **06.09.2017**
(21) Anmeldenummer: 17157885.9
(22) Anmeldetag: 24.02.2017
(51) Int. Cl.: A61F 2/24, A61L 15/28, A61L 26/00, A61L 27/20

(54) **REDUKTION VON PARAVALVULÄRER LECKAGE DURCH KONTROLLIERTEN THROMBUSAUFBAU**

(30) Priorität: 03.03.2016 DE 102016103843
(71) Anmelder: Biotronik AG, 8180 Bülach (CH)
(72) Erfinder: Quint, Bodo, 79802 Dettighofen (CH)
(74) Vertreter: Randoll, Sören

(57) **Zusammenfassung**

Die Erfindung betrifft eine Herzklappenprothese (1), aufweisend eine Herzklappe (2), und einen Grundkörper (3) zum Tragen der Herzklappe (2), wobei die Herzklappe (2) ein Abschlussmaterial (10) aufweist, das dazu ausgebildet ist, eine paravalvuläre Leckage durch Thrombusaufbau zumindest teilweise zu verschließen.

## Beschreibung

Die vorliegende Erfindung betrifft eine Herzklappenprothese umfassend eine Vorrichtung zum kontrollierten Thrombusaufbau.

Eine derartige Herzklappenprothese weist eine Herzklappe auf, die eine Herzklappe des Patienten ersetzen soll, sowie einen Grundkörper (in der Regel in Form eines Stents), der zum Tragen der Ersatzherzklappe dient, wobei jene Ersatzherzklappe an dem Grundkörper befestigt ist.

Solche Prothesen werden z.B. im Rahmen einer Transkatheter-Aortenklappenimplantation verwendet (auch TAVI genannt, für transcatheter aortic valve implantation). Hierbei stellt die paravalvuläre Leckage, bei der Blut zwischen der Gefäßwand und der eingesetzten Prothese hindurch strömt, eine der schwerwiegenden Komplikationen dar, die die prozedurale Effektivität minimalinvasiver Herzklappen beschränkt.

Hiervon ausgehend liegt der vorliegenden Erfindung die Aufgabe zugrunde, eine Herzklappenprothese bereitzustellen, die dem Risiko einer paravalvulären Leckage entgegen wirkt.

Diese Aufgabe wird durch eine Herzklappenprothese mit den Merkmalen des Anspruchs 1 gelöst. Vorteilhafte Ausgestaltungen der Erfindung sind in den Unteransprüchen angegeben und werden nachfolgend beschrieben.

Gemäß Anspruch 1 ist danach eine Herzklappenprothese vorgesehen, mit einer Herzklappe, bei der es sich vorzugsweise um eine künstliche Herzklappe aus einem biologischen Gewebe handelt, einem Grundkörper zum Tragen der Herzklappe, wobei die Herzklappe an dem Grundkörper befestigt ist, wobei die Herzklappe vorzugsweise mit dem Grundkörper vernäht ist. Erfindungsgemäß ist nun vorgesehen, dass die Herzklappenprothese zur Vermeidung einer paravalvulären Leckage der Herzklappenprothese an einer Außenseite des Grundkörpers ein flüssigkeitsdurchlässiges Abschlussmaterial aufweist, das dazu ausgebildet ist, bei Kontakt mit Blut eines Patienten einen Thrombus zu erzeugen, der eine paravalvuläre Leckage zumindest teilweise, vorzugsweise vollständig, verschließt bzw. abdichtet. Das Abschlussmaterial ist dabei vorzugsweise als Fasermaterial ausgebildet, das dabei dadurch charakterisiert ist, dass es eine Vielzahl an Fasern aufweist. Das Abschlussmaterial kann aber auch zum Beispiel in Form eines flüssigkeitsdurchlässigen schwammartigen Gebildes ausgeformt sein, an dem sich nach Einsetzen der Herzklappenprothese in den Patienten kontrolliert ein Thrombus bildet.

Die Erfindung betrifft also mit anderen Worten einen Mechanismus für einen systematischen Volumengewinn an natürlichem Dichtmaterial durch Thrombusformation und insbesondere auch dessen räumliche Begrenzung. Hierdurch können Zwischenräume, über die ansonsten paravalvuläre Leckagen stattfinden, verschlossen werden, ohne dass das hierfür benötigte Volumen an Dichtmaterial systematisch als Teil des Implantats geliefert werden muss. Dies hat insbesondere bei Einführung der Herzklappenprothese an den Implantationsort den bedeutenden Vorteil, dass das Volumen der kollabierten, nicht expandierten Herzklappenprothese äußerst gering gehalten werden kann, da das Volumen des Dichtmaterials nahezu eingespart ist, denn das Abschlussmaterial nimmt lediglich einen Bruchteil des Volumens ein, das durch gängige Dichtmaterialien verbraucht wird. Die Reduzierung des Volumens der einzuführenden Herzklappenprothese ist eines der bedeutenden Herausforderungen bei der Entwicklung einer künstlichen Herzklappe und der vorliegende Vorschlag liefert dazu einen eleganten Lösungsansatz.

Gemäß einer bevorzugten Ausführungsform der Erfindung ist vorgesehen, dass das Abschlussmaterial, bevorzugt in Form eines Fasermaterials, zumindest einen der folgenden Stoffe aufweist oder durch zumindest einen der folgenden Stoffe gebildet ist: ein Polymer, ein biologisches Polymer, ein künstliches Polymer, ein kationisches Polymer, ein Polykation, insbesondere biologischen Ursprungs, Chitosan, Chitosan Chlorid, Chitin.

Hierbei sind insbesondere polykationische Oberflächen von Interesse, die auf Compounds (Gemische oder auch Blends) mit Diethylaminoethyl-Dextran oder Diethylaminoethylierter Cellulose beruhen oder direkt durch Diethylaminoethylierung hydroxylreicher Polymeroberflächen hergestellt wurden. Geeignete kationische Polymere synthetischen Ursprungs können ausgewählt werden aus der Gruppe bestehend aus oder umfassend Reaktionsprodukte aus Dimethylamin-Polyamin-Epichlorhydrin, Polydimethyldiallylammoniumchlorid und quarternäre Derivate von Polydimethylaminoethylmethylacrylat. Vorzugsweise wird für das Abschlussmaterial ein Stoff ausgesucht, der unabhängig von einer Antikoagulationsmedikamentierung durch seine Oberflächeneigenschaften die aktive Erzeugung des besagten Thrombus ermöglicht und durch geeigneten morphologischen Aufbau diesen Thrombus mechanisch stabilisiert und/oder immobilisiert. Dies hat den besonderen Vorteil, dass der beabsichtigte Thrombusaufbau zur Abdichtung paravalvulärer Leckagen trotz routinemäßig verabreichter Antikoagulationsmedikamentierung gelingt, so dass eine Herzklappenprothese mit minimalem Volumen und integrierter Vorrichtung zum Abdichten paravalvulärer Leckagen bereitgestellt wird, die keine Änderung oder spezielle Einstellung üblicher Medikamentierung erfordert. Dies wird vorzugsweise durch ein kationisches Polymer und/oder ein Polykation, und weiter bevorzugt durch Chitosan erreicht.

Weiterhin ist gemäß einer bevorzugten Ausführungsform der Erfindung vorgesehen, dass das Abschlussmaterial in einem blutdurchlässigen Sack angeordnet ist, der an einer Außenseite des Grundkörpers angeordnet ist und am Grundkörper festgelegt ist. Der Sack kann hierbei insbesondere schlauchartig ausgebildet sein und an der Außenseite des Grundkörpers auf Höhe der Herzklappe verlaufen.

Vorzugsweise weist der Sack eine Hülle in Form eines Gewebes auf, die eine Filterstruktur aufweist, mit Durchgangsöffhungen oder Poren, die einen Durchmesser aufweisen, der signifikant größer ist als 10 µm, um einen ungehinderten Zugang von Blut zu dem in dem Sack lose angeordneten Abschlussmaterial zu gewährleisten. Liegt das Abschlussmaterial in Form von Fasermaterial vor, ist der Durchmesser der Fasern vorzugsweise größer als die oben genannte Filterstruktur bzw. der Durchmesser der besagten Durchgangsöffnungen/Poren. Vorzugsweise wird durch den Polykationeffekt eine Thrombusbildung in dem Sack an dem Abschlussmaterial erzeugt, wobei das umgebende Gebilde das eingeschlossene Volumen für das Polykation, bzw. den eingeschlossen Thrombus räumlich begrenzt.

Gemäß einer bevorzugten Ausführungsform weist der Sack bzw. dessen Hülle einen der folgenden biokompatiblen, sowie chemisch und mechanisch sehr stabilen Stoffe auf oder besteht aus einem der folgenden Stoffe: Polyethylen, und zwar vorzugsweise HDPE ("high density polyethylene") oder UHMWPE ("ultra-high-molecular-weight polyethylene") und Polyethylenterephthalat (PET). Des Weiteren sind auch resorbierbare Varianten bestehend aus oder umfassend Polyethylensuccinat, Polybutylensuccinat, Polyester, Polylactid oder Polycaprolacton geeignet.

Eine weitere prinzipielle Ausführungsform der Erfindung sieht vor, das Abschlussmaterial nicht in einem durch eine Hülle eines Sackes umgebenen Innenraum anzuordnen, sondern eine direkte Freisetzung des Materials (insbesondere Polykationfasern oder Polykationschwamm) um die Herzklappenprothese herum, um einen Volumengewinn durch Thrombusaktivierung zu nutzen. Es wird davon ausgegangen, dass im Falle einer Implantation die antikoagulative Medikamentierung in jedem Falle ausreichend ist, um eine Eskalation der Gerinnungskaskade zu vermeiden und die morphologische Struktur genügend Wechselwirkungen bietet, um den gebildeten Thrombus zu immobilisieren. In einer Ausführungsform werden die Fasern aus einem Compound hergestellt, der zu einem Teil auf einer rigiden Polymermatrix, zum anderen Teil auf einem kationischen Polymer beruht. Der kationische Polymeranteil kann dabei entweder aus einer vernetzten Variante eines ansonsten wasserlöslichen kationischen Polyelektrolyten oder aus einem kationischen Polymer, welches nur begrenzt durch Feuchtigkeit quellbar ist, gefertigt sein. Das beschriebene Compound bildet dabei vorteilhafterweise unterschiedliche Polymerphasen aus, welche unter Feuchtigkeitseinwirkung zu Spannungen in der Faser und zu einer Kräuselung der Faser führen.

Eine weitere Ausführungsform umfasst die Variante die Polykationfasern mittels eines Elektrospinning Prozesses zu erzeugen. Hierbei wird Chitosan direkt oder im Gemisch mit anderen Polymeren (bevorzugt PEO - Polyethylenoxid) aus saurer Lösung zu vliesartigen Strukturen ausspinnen. Bereits kommerziell verfügbare technische Umsetzungen des Elektrospinningprozesses erlauben die Ausführung des Elektrospinningprozesses mit dem simultanen Ausspinnen mehrerer, unterschiedlicher Fasertypen. Des Weiteren ist auch eine Umschaltung auf unterschiedliche Fasertypen innerhalb des Elektrospinningprozesses möglich. Mit dieser Prozessvariante lassen sich somit die Polykationfasern und eine umgebende Hülle, welche aus rigideren Stützfasern gebildet wird, innerhalb eines Verfahrensschrittes erzeugen.

Gemäß einer weiteren Ausführungsform der Erfindung ist bevorzugt vorgesehen, dass das Abschlussmaterial, bevorzugt in Form von Fasermaterial, an einer Außenseite des Grundkörpers am Grundkörper befestigt ist.

Weiterhin ist gemäß einer weiteren Ausführungsform der Erfindung vorgesehen, dass das Abschlussmaterial in Form vom Fasermaterial Multifilamente mit je einer Mehrzahl an miteinander verbundenen Fasern (bzw. Filamenten) aufweist. In einer Ausführungsform ist das Fasermaterial oder die Multifilamente bzw. Fasern am Grundkörper stoff-, kraft-, und/oder formschlüssig befestigt, z.B. durch Aufknoten der Multifilamente oder Fasern am Grundkörper oder durch Festkleben der Multifilamente oder Fasern am Grundkörper. Dabei können die Fasern unterschiedlichen Typs sein, um z.B. nichtresorbierbar langzeitstabil die mechanische Assoziation des Volumens zu gewährleisten. Weiterhin können die Fasern aus einem z.B. resorbierbaren chitosanhaltigen oder -basierten Polymer bestehen, welches zeitlich begrenzt die Thrombusbildung unterstützt.

Die Multifilamente bzw. deren Fasern weisen vorzugsweise die Eigenschaft auf, dass sie durch den (mit dem Herzschlag) wechselnden Blutfluss mechanisch vereinzelt werden und dadurch ihre Oberfläche vergrößern und somit die Thrombusbildung verstärken. Ferner bieten Multifilamente den Vorteil einer hohen Oberfläche bei geringem zusammengepresstem Volumen. Durch die Ausführung als Multifilamente wird somit ein schneller kontrollierter Thrombusaufbau unterstützt, während das Volumen bei der Einführung der Herzklappenprothese an den Implantationsort gering ist. In einer weiteren Ausführungsform werden die Multifilamente vor dem Implantieren mit einer Schlichte versehen. Eine Schlichte wie hierin beschrieben betrifft eine Beschichtung der Multifilamente, die einer Imprägnierung entspricht. Eine solche kann Schlichte kann zum Beispiel durch Aufsprühen oder Eintauchen aufgetragen werden. Vorteilhaft wirkt sich eine Schlichte auf den Multifilamenten in der Form aus, dass die Multifilamente kompakt, aber geschmeidig an den Implantationsort geführt werden können und dort schnell im Blutstrom besser separieren. Geeignete Schlichtmittel umfassen grundsätzlich im wässrigen Medium lösliche, biologisch verträgliche polymere Beschichtungsmittel und bevorzugt Polyethylenglykol verschiedener Größen, jedoch bevorzugt im Bereich von PEG-400 bis PEG-800, Polyvinylpyrrolidon, vorzugsweise mit einem K-Wert im Bereich von K17 bis K30, Hyaluronsäure und Polyzucker, insbesondere Pullulan oder Dextrane oder Gemische aus solchen Verbindungen. Durch die Einführung einer Schlichte kann zudem besser erreicht werden, dass die Thrombusbildung erst am Implantationsort einsetzt. Dadurch wird ein optimales Einsetzen der Prothese sicherer ermöglicht.

Dieser Effekt kann durch die Kombination mit z.B. quellenden oder schrumpfenden Segmenten in der Faser (z.B. Kräuseln der Faser) positiv verstärkt werden. Quellend wäre z.B. die Erzeugung der Fasern aus einem Polymergemisch, welches aus Chitosan und einem nicht löslichen, aber biologisch unbedenklichen Polymer wie z.B. Vinylpyrrolidonco-vinylacetat (P(VP-co-VAc)) besteht oder solche Polymere umfasst. Ausgehend davon, dass sich die unterschiedlichen Polymere untereinander nicht gegenseitig lösen, wird eine Faser erzeugt, die im trockenen Zustand gerade sein kann, sich aber durch Wasseraufnahme zufällig mechanisch verformt, beispielsweise aufkräuselt. In diesem Falle wird die Matrix der Faser aus einem kationischen Polymer gebildet. Wasserlösliche kationische Polymere wie zum Beispiel das oben angeführte Diethylaminodextran können mittels der Beimischung eines Polyanions (zum Beispiel in Form von 1-20% Polyacrylsäure) oder durch chemische Vernetzung (zum Beispiel durch Kondensation während der schmelzenden Verarbeitung, durch Umsetzung mit mehrfunktionellen Isocyanaten (vorzugsweise Hexamethylendiisocyanat (HMDI), epoxidhaltigen Vernetzer oder durch Umsetzung mit Melamin oder Derivaten davon) in Anwesenheit von Feuchtigkeit in einen stabilen Hydrogelzustand überführt werden. In diesem Falle eignet sich die kationische quellbare Polymerkomponente dazu, die Thrombusbildung zu gewährleisten. Der rigide Faseranteil kann ferner zum Beispiel durch eine bioresorbierbare Polymermatrix, zum Beispiel aus Polycaprolacton oder Derivaten davon, gebildet werden.

Entsprechend sind gemäß einer Ausführungsform der Erfindung die einzelnen Fasern oder Filamente dazu ausgebildet, sich bei Kontakt mit Wasser und/oder Blut von einem längserstreckten bzw. geraden ersten Zustand in einen zweiten Zustand zu verformen, wobei die Fasern im zweiten Zustand eine Vielzahl an Krümmungen aufweisen, so dass die einzelnen Fasern im zweiten Zustand z.B. gekräuselt oder gewellt sind. Vorteilhafterweise wird dadurch eine Volumenzunahme des Fasermaterials bewirkt.

Weiterhin ist gemäß einer entsprechenden Ausführungsform der Erfindung bevorzugt vorgesehen, dass die besagten Fasern ein Polymergemisch aufweisen, wobei das Polymergemisch Chitosan und ein durch Wasser und/oder Blut nicht lösliches Polymer, vorzugsweise P(VP-co-VAc), aufweist. Die Verwendung von P(VP-co-VAc) ist insbesondere vorteilhaft, da P(VP-co-VAc) hydrogelen Charakter zeigt; das bedeutet, dass P(VP-co-VAc) in Wasser zwar nicht löslich ist, unten diesen Bedingungen aber klebrig bleibt. In einer besonderen Ausführungsform kann das Polymergemisch auch einen geringen Anteil (bis 10%, vorzugsweise bis 5%) eines Polyanions aufweisen, z.B. Polyacrylsäure. Die Anwesenheit einer Mindermenge eines Polyanions hat den Vorteil, dass das Polymergemisch dadurch besser durch die Wechselwirkung zwischen Polykation und Polyanion immobilisiert werden kann. In einer weiteren Ausführungsform wird das Polymergemisch durch Chitosan und oxidativ vernetztes Polyvinylpyrrolidon gebildet. Das oxidativ vernetzte Polyvinylpyrrolidon wird dabei vorteilhaft schmelzend oder aus Lösung in das Polymergemisch überführt. In einer weiteren Ausführungsform kann das Polymergemisch aus zwei unterschiedlichen Polyanionen gebildet sein, wobei eines der Polyanionen mit einem wasserlöslichen Polymer gegraftet / derivatisiert ist.

Wie bereits oben beschrieben kann die Freisetzung der Fasern des Abschlussmaterials zusätzlich durch eine Ausrüstung der Fasern, insbesondere in Form einer Beschichtung in Form einer Schlichte, mit einem sich im biologischen System neutral verhaltenden sowie insbesondere biologisch abbaubaren, löslichen Polymer gesteuert werden. Hierdurch kann effektiv das Einsetzen des Thrombusaufbaus zeitlich verzögert werden. Bei dem biologisch abbaubaren, löslichen Polymer kann es sich z.B. um PVP (Polyvinylpyrrolidon), PEG (Polyethylenglykol), Polyzucker, insbesondere Pullulan, Dextrane und Hyaluronsäure handeln. In einer weiteren Ausführungsform sind die Fasern hydrophob ausgestaltet, wodurch eine Diffusion von Wasser/Blut immer noch möglich, aber deutlich verlangsamt ist. Mit dieser Ausführungsform wird weiterhin verhindert, dass die Fasern mit dem Schlauchelement des Einführsystems, welcher zur Freisetzung zurückgezogen wird, des Einführsystems verkleben.

Gemäß einer weiteren Ausführungsform der Erfindung ist vorgesehen, dass der Grundkörper der Prothese dazu konfiguriert ist, expandiert zu werden (z.B. in bekannter Weise durch einen Ballon oder durch Verwendung eines selbstexpandierenden Materials für den Grundkörper), um insbesondere die Herzklappenprothese am Implantationsort in einen bestimmungsgemäßen Zustand zu expandieren.

Gemäß einer alternativen Ausführungsform der Erfindung ist vorgesehen, dass der Grundkörper selbstexpandierbar ausgebildet ist. Hier ist der Grundkörper vorzugsweise aus einem Material geschaffen, das eine automatische Expansion des Grundkörpers ermöglicht, sobald dieser entsprechend freigegeben wird (z.B. aufgrund einer elastischen, insbesondere superelastischen Eigenschaft des Grundkörpers).

Besonders bevorzugt ist die Herzklappenprothese dazu konfiguriert per Katheter in ein Körperlumen eines Patienten implantiert zu werden, vorzugsweise mittels TAVI. Entsprechend betrifft ein weiterer Aspekt der Erfindung ein System mit einer erfindungsgemäßen Herzklappenprothese und einer Kathetereinrichtung, die zum Implantieren der Herzklappenprothese konfiguriert ist.

Weitere Merkmale und Vorteile der Erfindung sollen nachfolgend anhand von Figurenbeschreibungen von Ausführungsformen der Erfindung beschrieben werden. Es zeigen:
- Fig. 1: eine schematische Draufsicht auf eine erfindungsgemäße Herzklappenprothese in der Ausführungsform mit einem Sack zur Aufnahme eines Abschlussmaterials,
- Fig. 2: eine schematische, ausschnitthafte sowie teilweise geschnittene Ansicht der Prothese gemäß Figur 1; und
- Fig. 3: eine schematische Draufsicht einer weiteren erfindungsgemäßen Herzklappenprothese in der Ausführungsform ohne Sack mit Abschlussmaterial in Form von Fasermaterial.

### Beispiel 1:

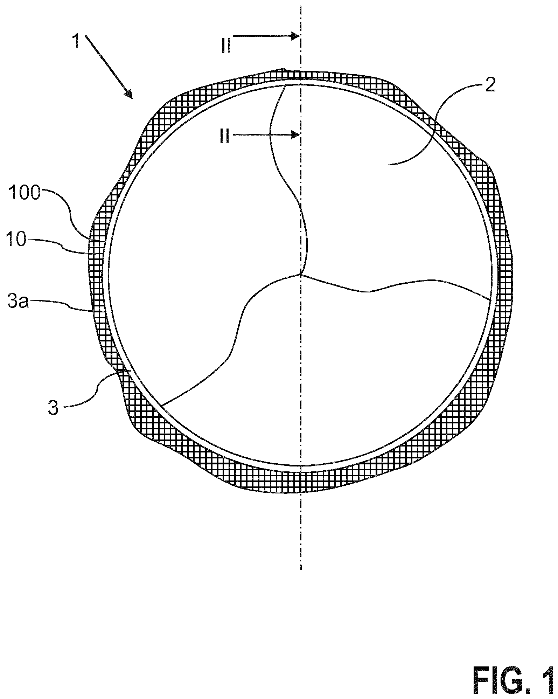

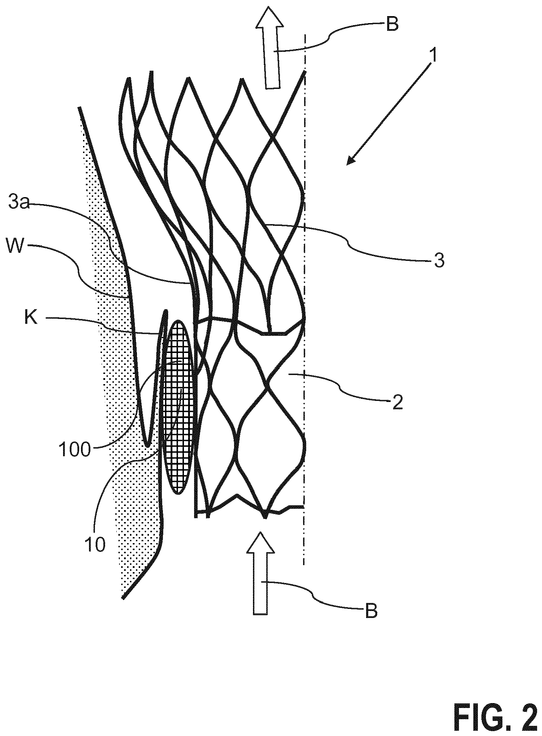

Gemäß Figuren 1 und 2 weist die Herzklappenprothese 1, eine vorzugsweise künstliche Herzklappe 2, bevorzugt aus einem biologischen Gewebe, auf, sowie einen Grundkörper 3 in Form eines expandierbaren oder selbstexpandierbaren Stents (siehe auch oben), der dazu konfiguriert ist, die Herzklappe in einem vom Grundkörper umgebenen Innenraum aufzunehmen, wobei die Herzklappe 2 an dem Grundkörper 3 befestigt ist, z.B. durch Vernähen der Herzklappe 2 mit dem Grundkörper 3.

Im implantierten, expandierten Zustand kann die Herzklappenprothese dazu konfiguriert sein, z.B. eine defekte Aortenklappe K eines Patienten zu verdrängen und funktionell zu ersetzen. Das Blut strömt dann in der Richtung B durch die am expandierten Grundkörper festgelegte Ersatzherzklappe 2, wobei die Prothese 1 umlaufend an der defekten Klappe K bzw. an der umgebenden Gefäßwand W abdichtend anliegen soll. Hierbei soll insbesondere kein Blutfluss zwischen der Gefäßwand W bzw. Klappe K und der Prothese 1 stattfinden. Ein solcher Blutfluss stellt eine sogenannte paravalvuläre Leckage dar.

Die Herzklappenprothese 1 weist nun erfindungsgemäß zum Abdichten von solchen paravalvulären Leckagen an einer dem Innenraum des Grundkörpers abgewandten Außenseite 3a des Grundkörpers 3 ein Abschlussmaterial 10 auf, das dazu ausgebildet ist, bei Kontakt mit Blut eines Patienten der Herzklappenprothese 1 einen Thrombus zu erzeugen, der die paravalvuläre Leckage im Idealfall abdichtet. Hierbei ist gemäß den Figuren 1 und 2 vorgesehen, dass das Abschlussmaterial 10 in einem blutdurchlässigen Sack 100 angeordnet ist, der an der Außenseite 3a des Grundkörpers 3 angeordnet ist und am Grundkörper 3 befestigt ist, wobei der Sack 100 den Grundkörper 3 im Umfangsrichtung (also Quer zur Richtung B) umläuft.

Der Sack 100 bzw. eine Hülle des Sacks 100, die das Abschlussmaterial 10 einschließt, kann hierbei aus einem hochfesten, annähernd inerten Gewebe, vorzugsweise aus PET, gefertigt sein. Um keine unkontrollierte Thrombusformation zu erzeugen, kann das Gewebe mit einer aktiven Antikoagulanz zusätzlich ausgerüstet sein. Innerhalb des Gewebes befindet sich das Abschlussmaterial 10, z.B. in Form von textil immobilisierten Fasern, welche unabhängig von der Anwesenheit von Koagulantien eine Thombusformation auslösen. Diese Fasern bestehen z.B. aus einem unlöslichen Polykation, vorzugsweise aus Chitosan. Durch aktive Thrombusbildung wird insbesondere ein inflammatorischer Effekt erzeugt, der den Gewebeaufbau um die Prothese 1 fördert. Primär soll der durch den Thrombus erzeugte Volumengewinn Kavitäten um die Prothese 1 dauerhaft verschließen. Wenn das Risiko der Auslösung einer Gerinnungskaskade zu hoch ist, aber auch als präventive Maßnahme, kann das Gewebe des Sacks 100 selbst oder ein lokaler Bereich innerhalb des Sacks 100 mit Antikoagulantien ausgerüstet werden.

### Beispiel 2:

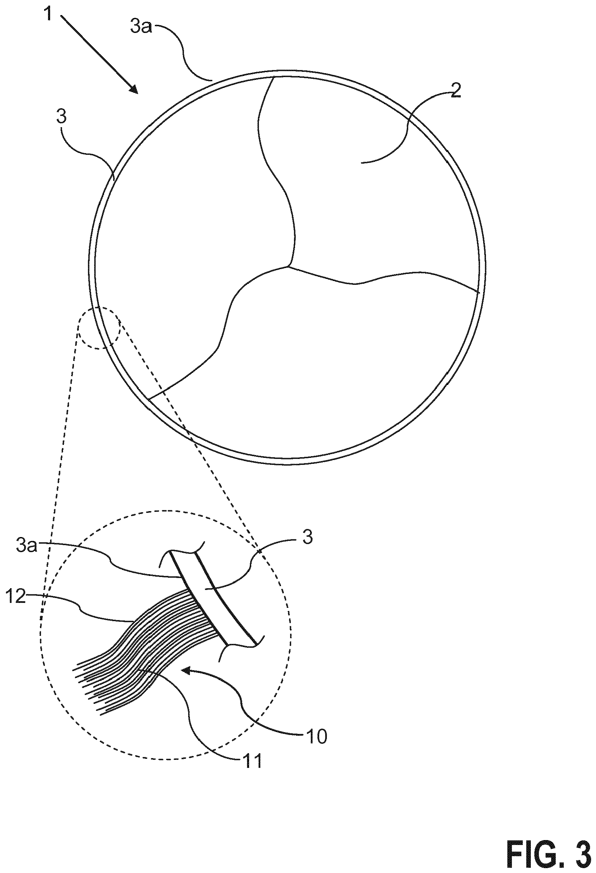

Figur 3 zeigt eine weitere Ausführungsform der Erfindung, bei die Herzklappenprothese 1 wie oben beschrieben einen Grundkörper 3 sowie eine Herzklappe 2 aufweist. Im Unterschied zu den Figuren 1 und 2 ist hierbei vorgesehen, dass das Abschlussmaterial 10 am Grundkörper festgelegt ist, jedoch nicht von einem Sack 100 eingeschlossen ist, der ein Volumen der Thrombusformation begrenzt.

Gemäß dem Detail der Figur 3 weist das Abschlussmaterial 10 wiederum eine Mehrzahl an Fasern 12 auf, die an der Außenseite 3a des Grundkörpers 3 am Grundkörper 3 festgelegt sind. Hierbei ist vorzugsweise vorgesehen, dass die Fasern 12 Multifilamente 11 mit je einer Mehrzahl an miteinander verbundenen Fasern 12 bilden.

An der Außenseite 3a der Prothese 1 sind die Multifilamente, die z.B. aus Polykationfasern (z.B. Chitosanfasern) bestehen, aufgeknotet oder über eine Klebung oder einen Formschluss permanent am Grundkörper 3 fixiert.

Werden nun diese Multifilamente 11 dem wechselnden Blutfluss ausgesetzt, werden sie mechanisch vereinzelt, vergrößern dadurch ihre Oberfläche und verstärken somit die Thrombusbildung. Dieser Effekt kann durch die Kombination mit quellenden oder schrumpfenden Segmenten in der Faser (z.B. Kräuseln der Faser) positiv verstärkt werden. Quellend wäre z.B. die Erzeugung der Fasern aus einem Polymergemisch, welches aus Chitosan und einem nicht löslichen aber biologisch unbedenklichen Polymer wie z.B. einem P(VP-co-VAc) besteht. Entsprechend sind die Fasern 12 im trockenen Zustand gerade bzw. längserstreckt ausgebildet, verformen sich aber durch Wasseraufnahme in zufälliger Weise.

Die Freisetzung der Fasern 12, vorzugsweise durch Vereinzelung der Fasern, kann zusätzlich durch einer Ausrüstung der Fasern 12, z.B. einer Beschichtung der Fasern 12, mit einem sich im biologischen System neutral verhaltenden, insbesondere auch biologisch abbaubaren, löslichen Polymer (z.B. PVP, PEG - vorzugsweise aber Polyzucker wie z.B. Pullulan) gesteuert werden.

Polyzucker, wie z.B. Pullulan, werden hierbei bevorzugt eingesetzt, da diese durch intensive Trocknung (Dehydratation) reversibel vernetzt werden können und dadurch in ihrer Freisetzungsfunktion konfektionierbar sind.

Im Ergebnis sind die erfindungsgemäßen Lösungen mit Vorteil dazu geeignet, regellose Kavitäten um die Herzklappenprothese 1 herum zu schließen, um einer paravalvulären Leckage entgegen zu wirken.

## Patentansprüche

1. Herzklappenprothese (1), aufweisend eine Herzklappe (2), einen Grundkörper (3) zum Tragen der Herzklappe (2), wobei die Herzklappe (2) an dem Grundkörper (3) befestigt ist, **dadurch gekennzeichnet,**
**dass** die Herzklappenprothese (1) an einer Außenseite (3a) des Grundkörpers (3) ein Abschlussmaterial (10) aufweist, das dazu ausgebildet ist, einen Thrombus zu erzeugen.

2. Herzklappenprothese nach Anspruch 1, **dadurch gekennzeichnet, dass** das Abschlussmaterial (10) zumindest einen der folgenden Stoffe aufweist oder durch zumindest einen der folgenden Stoffe gebildet ist: ein Polymer, ein biologisches Polymer, ein künstliches Polymer, ein kationisches Polymer, ein Polykation, Chitosan.

3. Herzklappenprothese gemäß Anspruch 1 oder 2, **dadurch gekennzeichnet, dass** das Abschlussmaterial (10) am Grundkörper (3) befestigt ist oder in einem blutdurchlässigen Sack (100) angeordnet ist, der an der Außenseite (3a) des Grundkörpers (3) angeordnet und am Grundkörper (3) befestigt ist.

4. Herzklappenprothese nach Anspruch 3, **dadurch gekennzeichnet, dass** der Sack (100) zumindest einen der folgenden Stoff aufweist oder aus zumindest einem der folgenden Stoff besteht: Polyethylen, Polyethylenterephthalat, Polyethylensuccinat, Polybutylensuccinat, Polyester, Polylactid, Polycaprolacton.

5. Herzklappenprothese nach einem der vorhergehenden Ansprüche, **dadurch gekennzeichnet, dass** das Abschlussmaterial (10) eine Mehrzahl an Fasern aufweist.

6. Herzklappenprothese nach Anspruch 5, **dadurch gekennzeichnet, dass** die Fasern Multifilamente (11) mit je einer Mehrzahl an miteinander verbundenen Fasern bilden.

7. Herzklappenprothese nach einem der Ansprüche 1 bis 3, 5 oder 6, **dadurch gekennzeichnet, dass** die Fasern (12) oder die Multifilamente (11) am Grundkörper (3) stoff-, kraft- und/oder formschlüssig festgelegt sind.

8. Herzklappenprothese nach einem der vorhergehenden Ansprüche, **dadurch gekennzeichnet, dass** die einzelnen Fasern dazu ausgebildet sind, sich bei Kontakt mit Wasser von einem längserstreckten ersten Zustand in einen zweiten Zustand zu verformen, wobei die Fasern im zweiten Zustand einer Vielzahl an Krümmungen aufweisen.

9. Herzklappenprothese nach einem der vorhergehenden Ansprüche, **dadurch gekennzeichnet, dass** die Fasern ein Polymergemisch aufweisen, wobei das Polymergemisch Chitosan und ein wasserunlösliches Polymer, vorzugsweise P(VP-co-VAc), aufweist.

10. Herzklappenprothese nach einem der vorhergehenden Ansprüche, **dadurch gekennzeichnet, dass** die einzelnen Fasern mit einem löslichen Polymer ausgestattet, vorzugsweise beschichtet, sind.

11. Herzklappenprothese nach Anspruch 10, **dadurch gekennzeichnet, dass** das lösliche Polymer ausgesucht ist aus der Gruppe umfassend oder bestehend aus Polyvinylpyrrolidon, Polyethylenglykol, Hyaluronsäure und Polyzucker, insbesondere Pullulan oder Dextrane.

12. Herzklappenprothese nach einem der vorhergehenden Ansprüche, **dadurch gekennzeichnet, dass** der Grundkörper (3) expandierbar oder selbstexpandierbar ist.

13. Herzklappenprothese nach einem der vorhergehenden Ansprüche, **dadurch gekennzeichnet, dass** die Herzklappenprothese (1) per Katheter implantierbar ist.
